# EUROPEAN PATENT APPLICATION

(11) **EP 0 887 048 A1**
(43) Date of publication of application: **30.12.1998**
(21) Application number: 98117867.6
(22) Date of filing: 06.09.1994
(51) Int. Cl.: A61B 19/00, A61B 19/12

(54) **Apparatus for performing hand assisted minimally invasive surgery**

(30) Priority: 06.09.1993 IE 930649
(62) Divisional of application: 94924983.3
(71) Applicant: Encoret Limited, Bray, County Wicklow (IE)
(72) Inventor: Bonadio, Frank, Bray, County Wicklow (IE)
(74) Representative: McCarthy, Denis Alexis

(57) **Abstract**

An apparatus for performing hand assisted, minimally invasive surgery comprises a sleeve (2) having an entry opening (4) at an outer end thereof and an exit opening (5) at an inner end thereof to access a patient's body. The sleeve (2) is a flexible gas-impermeable material. Exit gas sealing means (10) are provided for attaching to, and sealing the exit opening (5) to the patient's body to provide a gas pressure tight seal with the patient's body. Entry gas sealing means (20) are provided for sealing the entry opening (4) to provide a pressure tight seal about a surgeon's or physician's arm passing therethrough to create a controlled pressurised gas environment within the sleeve (2). The entry gas sealing means comprises a valve means (20) through which the surgeon passes his or her arm. The apparatus is attachable to the patient's body with the exit opening adjacent an incision in the patient's body and a surgeon's or physician's arm can be positioned in the sleeve (2) with the entry opening (4) substantially gas sealed therearound by the valve means (20) so that the apparatus provides passage into the interior of the patient's body to permit the surgeon to reach into said interior of the body to perform a surgical procedure within the gas pressurised body cavity.

## Description

The invention relates to an apparatus for use in surgery and in particular to an apparatus to be used in minimal invasive surgery in which surgery is carried out by making the minimum number of incisions in a patient's body.

Abdominal surgery is generally carried out by making a very large incision allowing a surgeon to enter the body cavity with both hands. Such surgery is traumatic for the patient and the healing process is lengthy. Some laproscopic surgery such as hernia operations may be carried out by surgeons using minimal invasive techniques with trocar assemblies. However, the techniques are generally complex and difficult and are not widely used.

Accordingly, the invention provides an apparatus for performing hand assisted, minimally invasive surgery comprising a sleeve having an entry opening at an outer end thereof; and an exit opening at an inner end thereof to access a patient's body; characterised in that: the sleeve comprises a flexible gas-impermeable material; exit gas sealing means are provided for attaching to, and sealing the exit opening to the patient's body to provide a gas pressure tight seal with the patient's body; entry gas sealing means are provided for sealing the entry opening to provide a pressure tight seal about a surgeon's or physician's arm passing therethrough to create a controlled pressurised gas environment within the sleeve; and the entry gas sealing means comprises a valve means through which the surgeon passes his or her arm; whereby the apparatus is attachable to the patient's body with the exit opening adjacent an incision in the patient's body and a surgeon's or physician's arm can be positioned in the sleeve with the entry opening substantially gas sealed therearound by the valve means so that the apparatus provides passage into the interior of the patient's body to permit the surgeon to reach into said interior of the body to perform a surgical procedure within the gas pressurised body cavity.

Advantageously, the valve means is of a material which is sufficiently flexible to allow an arm to be passed therethrough and to form a gas seal about the arm when passed therethrough.

Conveniently, the valve means comprises a first mounting in the sleeve entry opening, a second mounting and a sealing body of flexible material extending between the mountings one of the mountings being twistable relative to the other to twist the sealing body into gas tight engagement with an arm passing therethrough.

Preferably, fixing means are provided for fixing one mounting relative to the other in the gas sealing position.

Advantageously, the fixing means comprises inter-engaging formations provided on the mountings.

The first mounting may comprise a ring mounted in the sleeve at the entry opening thereof and the second mounting may comprise a ring to which the sealing material is attached.

Advantageously, the sleeve comprises a generally cylindrical body closed at one end thereof and the exit opening is provided in a side wall of the sleeve adjacent the closed end; optionally the exit gas sealing means comprises a flange around the exit for sealing against the body of a patient; optionally the flange is provided with an adhesive for adhering to the patient's body; optionally the exit and flange are covered by a peel-off cover; and optionally the exit opening includes a mounting ring adapted to surround an incision in the patient's body.

Conveniently, the sleeve includes at least one additional entry opening which permits another arm or an instrument to be passed into the sleeve while said entry opening has an arm positioned therein.

Additionally, the sleeve is provided with a sealing cuff disposed between said entry and exit openings for substantially sealing the sleeve when said entry opening is in an open condition.

The invention will be more clearly understood from the following description thereof, given by way of example only with reference to the accompanying drawings in which: -
Fig. 1 is a plan view of a sleeve;
Fig. 2 is a cross-sectional view of the sleeve of Fig. 1;
Fig. 3 is a plan view of a sleeve with an entry sealing means in position;
Fig. 4 is a perspective view of the sleeve of Fig. 3 is use;
Fig. 5 is a perspective view of the entry sealing means used in the sleeve of Figs. 3 and 4 in an open configuration;
Fig. 6 is a side elevational view of the sealing means of Fig. 5 in an intermediate position;
Fig. 7 is a perspective view of the sealing means of Fig. 5 in a sealed configuration;
Fig. 8 is a perspective view of another sleeve according to the invention; and
Fig. 9 is a side cross-sectional view of a further sleeve according to the invention.
Fig. 10 is a side cross-sectional view of one further sleeve according to the invention and
Fig. 10a is a rear end view of the sleeve shown in Fig. 10;

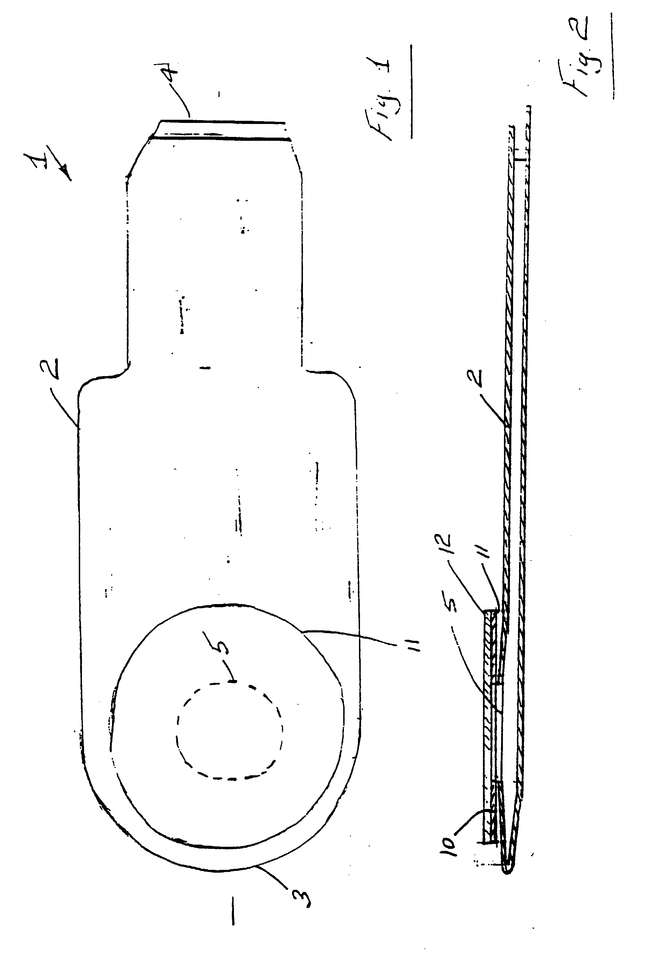

Referring to the drawings and initially to Figs. 1 to 7 thereof, there is illustrated an apparatus for use in surgery according to the invention indicated generally by the reference numeral 1. The apparatus 1 comprises a sleeve 2 of flexible gas-impermeable material. The sleeve 2 in this case comprises a generally cylindrical body closed at one end 3 thereof and open at the other end 4 thereof to define an entry opening at an outer end for passage of an instrument and/or surgeon's arm. An exit opening 5 is provided in a side wall of the sleeve 2 as illustrated particularly in Fig. 2 to provide an access point for entering a patient's body through an incision therein.

Exit sealing means 10 for sealing the exit opening 5 to a patient's body is in this case provided by a flange 11 around the exit opening 5 to the outer face of which is applied a pressure sensitive adhesive for adhering to the body of the patient. The adhesive side of the flange 11 is covered prior to use with a peel-off cover 12.

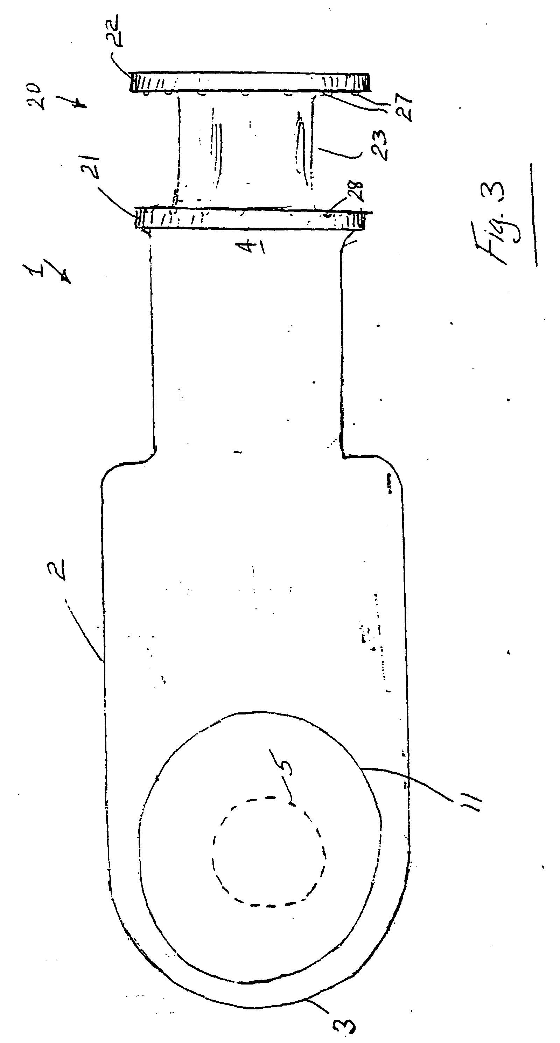

Entry sealing means which for clarity is not illustrated in Figs. 1 and 2 is in this case provided by a valve means indicated generally by the reference numeral 20 and illustrated particularly in Figs. 3 to 7. The valve means 20 comprises a first mounting provided by a ring 21 attached to the body of the sleeve 2 at the entry 4 and a second mounting provided by another ring 22 which is attached to the first ring 21 by a sealing member 23 of flexible material extending between the rings.

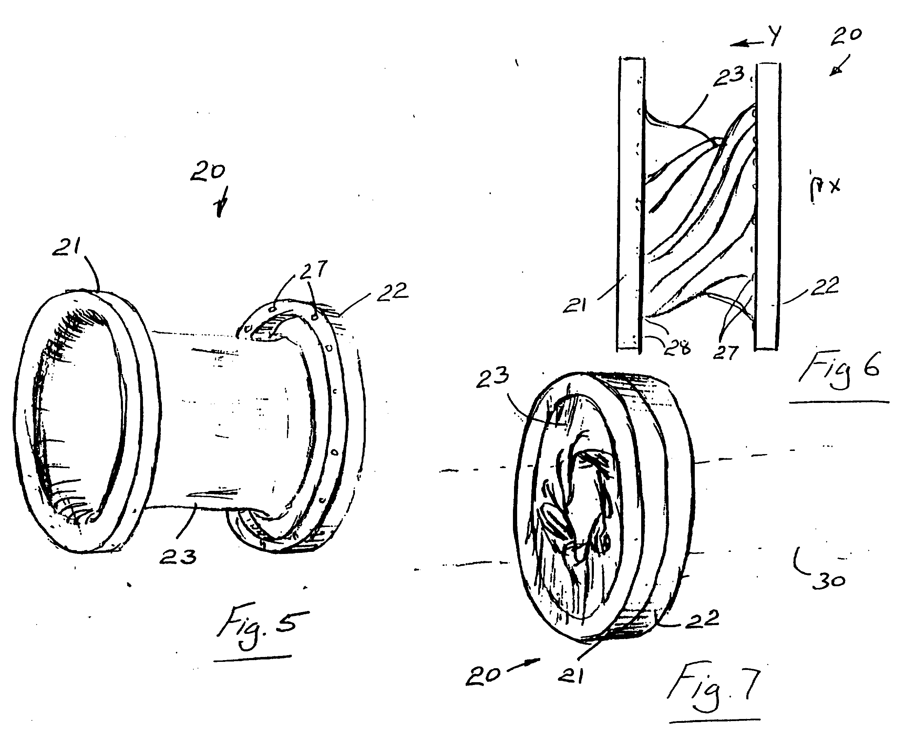

The outer ring 22 with the flexible body 33 attached is rotated to twist the sealing body 23 in the direction of the arrow X illustrated in Fig. 6 to engage and seal against a surgeon's arm 30 passing therethrough. When the sealing member 23 is in sealing engagement the outer ring 22 is pushed forwardly in the direction of the arrow Y against the inner ring 21 and the rings are engaged together to maintain the sealing engagement.

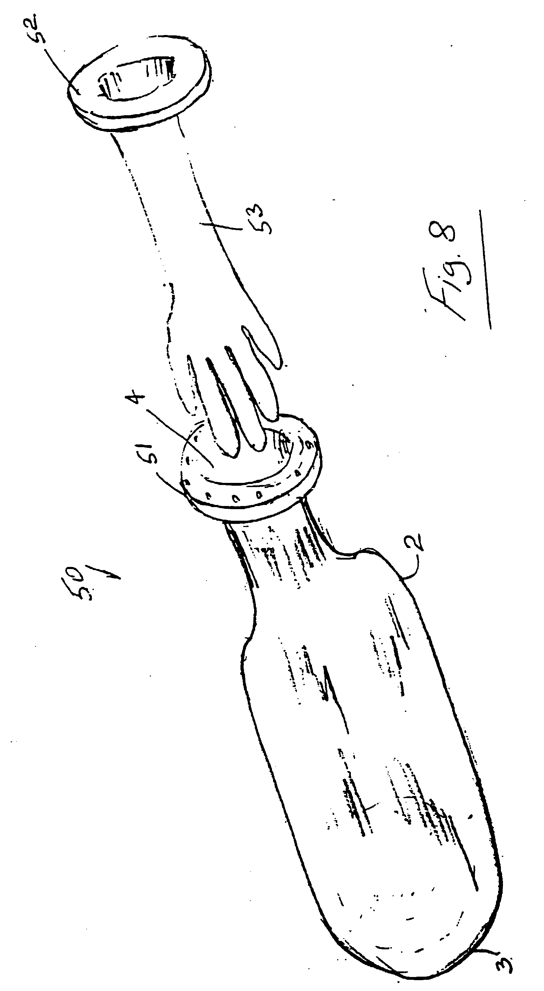

Fixing means for preventing rotation of the rings 21, 22 relative to one another when the rings are in the sealing position illustrated in Fig. 7 is in this case provided by a plurality of projections 27 on one of the rings 22 which are engageable with a plurality of complimentary shaped recesses 28 in the other rings 21 to lock the rings 21, 22 against rotation in the sealing position.

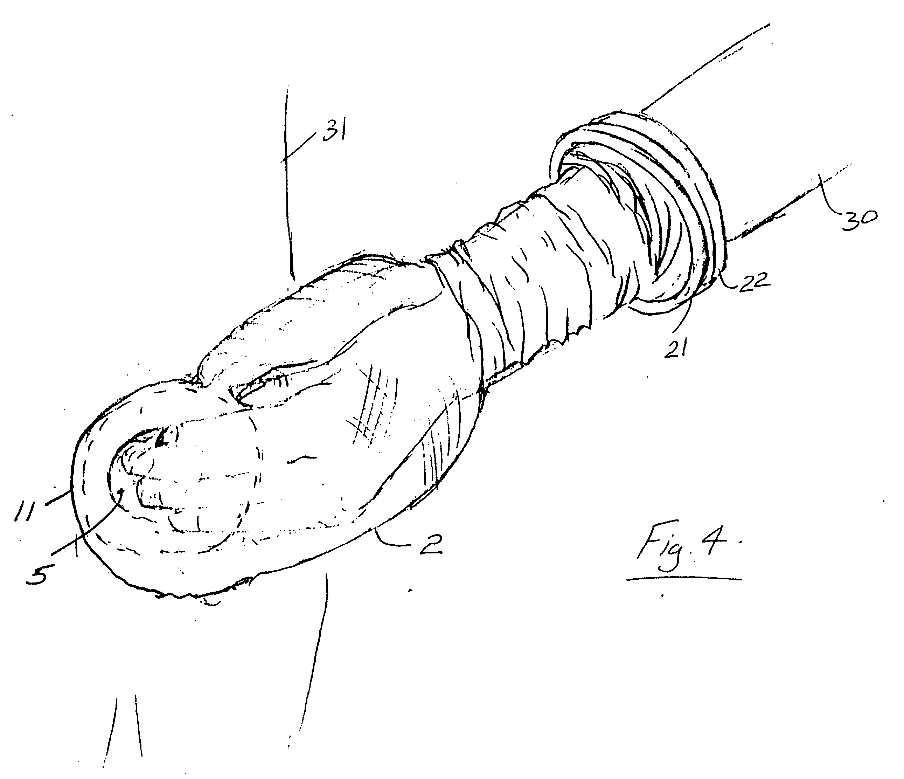

In use, an incision is first made in a patient 31. The cover 11 is then removed and the flange 12 is adhesively bonded to the patient around the incision as illustrated particularly in Fig. 4. The sleeve is arranged so that the exit opening 5 is aligned with the incision in the patient 31. With the entry sealing means 20 in the open non-sealing configuration illustrated in Figs. 3 and 5 a surgeon passes his hand and arm 30 through the entry 4 and the exit opening 5 to enter the patient's body through the incision. When the surgeon's arm 30 has passed through the sealing means 20 a desired distance, the outer ring 22 with the sealing body 23 attached is rotated to twist the sealing body 23 to engage against the surgeon's arm 30 until a relatively tight seal is obtained. The ring 22 is then pushed forwardly against the ring 21 and the projections are engaged in the recesses 28 to lock the rings 21, 22 together against rotation in the sealing configuration. In the case of bowel re-section surgery, gas is pumped into the patient's body cavity where the surgery is to be performed, the gas exiting through the incision in the patient and the opening 5 into the sleeve 2 to create a controlled pressurised environment in the sleeve 2 in which the sleeve 2 is inflated. The surgeon carries out the surgery as required and when completed the ring 22 is released from the ring 21 and contra-rotated until the flexible body 23 is in the non sealing position allowing the surgeon to extract his hand through the sleeve 2.

There are many advantages of the invention. Because a surgeon need only make a relatively small incision the trauma to the patient is minimised, there is less risk of damage to the immune system and the healing time is short with a consequent decrease in the length of the hospital stay required. The techniques are considerably simpler than convention laproscopic surgical techniques and can be readily performed by a surgeon with minimal additional training. A wide range of operations can be performed using the apparatus of the invention.

Referring to Fig. 8 there is illustrated another apparatus for use in surgery according to the invention indicated generally by the reference numeral 50. The apparatus 50 is similar to the apparatus described above with reference to Figs. 1 to 4 and like parts are assigned the same reference numerals. In this case the entry sealing means comprises a first ring 51 mounted to the sleeve 2 at the entry 4 and a second separate ring 52 at the free end of a surgical glove 53. When a surgeon's arm with the glove 53 passes through the entry 5 the rings 51, 52 are arranged to sealingly engage to create a controlled environment within the sleeve 2 during an operation.

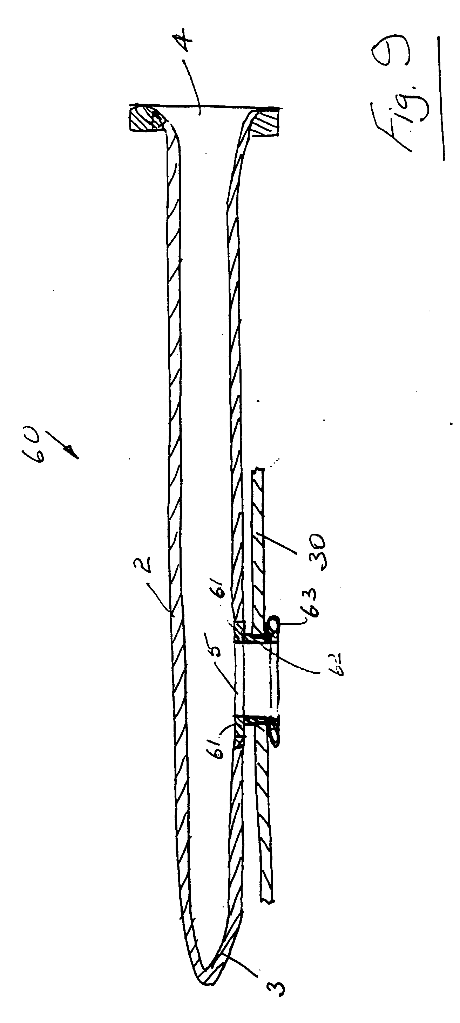

Referring to Fig. 9 there is illustrated a further apparatus according to the invention for use in surgery and indicated generally by the reference numeral 60. In this case, the exit sealing means comprises a sealing diaphragm having a first ring 61 attached to the sleeve 2 and a flexible diaphragm 62 extending from the ring 61 and terminating in an inner ring 63 which is inserted through the incision to engage with the body tissue 30 as illustrated. The sealing diaphragm seals the exit 5 of the sleeve 2 to the incision in the patient's body to create a controlled pressurised environment in the sleeve 2.

With reference to Figs. 10 and 10a, the sleeve 70 is an impermeable and flexible sleeve, one end of which is configured to provide a pressure tight seal against a surgeons forearm with the other end configured to provide a pressure tight seal with a patients skin. With the sleeve 70, and integral with it, is a non-return valve 74, in the form of a sealing cuff, permitting the removal of a surgeons hand and forearm from the sleeve whilst maintaining pressure within a patients abdomen.

The sleeve 70, in overall shape and method of construction, is substantially equivalent to known colostomy bags, differing in that its overall length is greater, there is the integral non-return valve 74 and the open end permits attachment to a surgeons forearm. The sleeve comprises two layers of medical grade polyethylene, one with an orifice 71, edge welded using a radio frequency (RF) welding technique. An annular flange 72 of medical grade polyethylene, one surface of which is coated with medical grade adhesive, is welded to the sleeve as shown. The non-return valve 74 comprises two layers of medical grade polyethylene welded to the sleeve to form the valve indicated. The arm attachment device consists of two rigid medical grade polyethylene circular members 76,77 connected, as indicated, by a thin walled cylindrical tube 78 of elastomer (medical grade latex or similar). Rotation of member 76 relative to 77 causes the latex tube to form an iris diaphragm effectively reducing the size of the arm access opening. Attachment of member 77 to the sleeve is achieved using medical grade adhesive.

The technique of attachment of this device to a patient is similar to that of a colostomy bag. The annular, adhesive coated flange is applied to a patients skin such that the annulus encircles the access incision. Adhesion may be assisted by the application of an adhesive surgical drape prior to making said incision. Attachment to a surgeons forearm is achieved by inserting a hand and forearm into the sleeve through member 76. Rotation of member 76 relative to member 77 causes an iris diaphragm to form effectively gripping the forearm with the elastomer sleeve. Further movement of the hand towards opening 71 causes the non-return valve 74 to open and access to the abdominal cavity is possible through the access incision. Removal of a surgeons hand is in the reverse order to that above with the result that the non-return valve 74 closes maintaining abdominal pressure.

It is anticipated that in some cases adhesive may be applied to a patient around the area of an incision to which a sealing ring of the sleeve is to be attached during preparations for an operation. Adhesive may alternatively or additionally be applied to the ring to be attached around the area of an incision. Either or both layers of adhesive may be covered by a sterile wrapping material through which the incision may be made. Either or both layers of adhesive may be provided with peel off covers.

It will further be appreciated that the sleeve may incorporate an air lock to facilitate changing of an instrument and/or debris such as cancer cells during an operation without breaking the sterilised environment in the sleeve.

The sleeve may be provided with more than one inlet opening for a surgeon's arms and/or instruments.

The sleeve may also be provided with means to create an intermediate pressurised environment by, for example, providing two inlet sealing cuffs spaced-apart along the sleeve. The inner of the cuffs being sealed before the seal provided by the outer cuff is opened.

The invention is not limited to the embodiments hereinbefore described, which may be varied in construction and detail without departing from the scope of the invention as defined in the appended claims.

## Claims

1. Apparatus for performing hand assisted, minimally invasive surgery comprising
a sleeve (2) having an entry opening (4) at an outer end thereof;
and an exit opening (5) at an inner end thereof to access a patient's body;
characterised in that:
the sleeve (2) comprises a flexible gas-impermeable material;
exit gas sealing means (10) are provided for attaching to, and sealing the exit opening (5) to the patient's body to provide a gas pressure tight seal with the patient's body;
entry gas sealing means (20) are provided for sealing the entry opening (4) to provide a pressure tight seal about a surgeon's or physician's arm passing therethrough to create a controlled pressurised gas environment within the sleeve (2);
and the entry gas sealing means comprises a valve means (20) through which the surgeon passes his or her arm;
whereby the apparatus is attachable to the patient's body with the exit opening adjacent an incision in the patient's body and a surgeon's or physician's arm can be positioned in the sleeve (2) with the entry opening (4) substantially gas sealed therearound by the valve means (20);
so that the apparatus provides passage into the interior of the patient's body to permit the surgeon to reach into said interior of the body to perform a surgical procedure within the gas pressurised body cavity.

2. Apparatus as claimed in Claim 1, wherein the valve means (20) is of a material which is sufficiently flexible to allow an arm to be passed therethrough and to form a gas seal about the arm when passed therethrough.

3. Apparatus as claimed in Claim 1 or Claim 2, wherein the valve means (20) comprises a first mounting (21) in the sleeve entry opening (4), a second mounting (22) and a sealing body (23) of flexible material extending between the mountings (21, 22), one of the mountings being twistable relative to the other to twist the sealing body (23) into gas tight engagement with an arm passing therethrough.

4. Apparatus as claimed in Claim 3, wherein fixing means are provided for fixing one mounting (21, 22) relative to the other in the gas sealing position.

5. Apparatus as claimed in Claim 4, wherein the fixing means comprises inter-engaging formations (27, 28) provided on the mountings.

6. Apparatus as claimed in any of Claims 3 to 5, wherein the first mounting comprises a ring (21) mounted in the sleeve (2) at the entry opening (4) thereof.

7. Apparatus as claimed in Claim 6, wherein the second mounting comprises a ring (22) to which the sealing material (23) is attached.

8. Apparatus as claimed in any one of the preceding claims, wherein the sleeve (2) comprises a generally cylindrical body closed at one end (3) thereof and the exit opening (5) is provided in a side wall of the sleeve (2) adjacent the closed end;
optionally wherein the exit gas sealing means (10) comprises a flange (11) around the exit (5) for sealing against the body of a patient;
optionally wherein the flange (11) is provided with an adhesive for adhering to the patient's body;
optionally wherein the exit (5) and flange (11) are covered by a peel-off cover (12);
and optionally in which the exit opening (10) includes a mounting ring (61, 62, 63) adapted to surround an incision in the patient's body.

9. Apparatus as claimed in any one of the preceding claims, wherein the sleeve (2) includes at least one additional entry opening which permits another arm or an instrument to be passed into the sleeve (2) while said entry opening (4) has an arm positioned therein.

10. Apparatus as claimed in any one of the preceding claims, wherein the sleeve (70) is provided with a sealing cuff (74) disposed between said entry and exit openings for substantially sealing the sleeve when said entry opening is in an open condition.
